# EUROPEAN PATENT SPECIFICATION

(11) **EP 2 579 860 B1**
(45) Date of publication and mention of the grant of the patent: **23.04.2014**
(21) Application number: 11726133.9
(22) Date of filing: 14.06.2011
(51) Int. Cl.: A61K 9/28, A61K 31/55, A61K 9/22, C07D 223/16

(54) **IVABRADINE-CONTAINING PHARMACEUTICAL COMPOSITION WITH MODIFIED RELEASE**
IVABRADINHALTIGE PHARMAZEUTISCHE ZUSAMMENSETZUNG MIT MODIFIZIERTER FREISETZUNG
COMPOSITION PHARMACEUTIQUE CONTENANT DE L'IVABRADINE A LIBERATION MODIFIEE

(30) Priority: 23.06.2010 IN CH17602010; 14.06.2010 EP 10165884; 14.06.2010 EP 10165881
(43) Date of publication of application: 17.04.2013
(73) Proprietor: Ratiopharm GmbH, 89079 Ulm (DE)
(72) Inventor: GIDWANI, Ramesh, Matioram, Maharasthra 400080 (IN); KOLHATKAR, Mayur, Vilas, Thane (W) 400610 (IN); MEERGANS, Dominique, 81379 München (DE); STEFAN, Ralph, 88370 Ebenweiler (DE); GEIER, Jens, 89610 Oberdischingen (DE)
(74) Representative: Teipel, Stephan
(86) International application number: PCT/EP2011/059864
(87) International publication number: WO 2011/157720

(56) References cited:
- WO-A1-2009/124940
- WO-A2-2008/065681
- US-A1- 2005 228 177
- US-A1- 2010 041 640
- DATABASE CAPLUS, [Online] 5 September 2007 (2007-09-05), ZHAO ZHIQUAN: "New formulations containing nitrates and ivabradine for treating myocardial ischemia", XP002609524, retrieved from CAPLUS Database accession no. 2007-1002734 -& CN 101 028 518 A (LUNAN PHARMACEUTICAL GROUP CO [CN]) 5 September 2007 (2007-09-05)

## Description

The invention relates to an oral administration form with modified release, containing (a) ivabradine adipate and a combination of (b) water-soluble excipient and (c) water-insoluble excipient; and a process for the preparation thereof. The present invention furthermore relates to the salt of ivabradine with adipic acid, and uses of the combination of a water-soluble excipient and a water-insoluble excipient.

Ivabradine has the chemical composition (S)-3-{3-[(3,4-dimethoxybicyclo[4.2.0]octa-1,3,5-trien-7-ylmethyl)methylamino]propyl}-7,8-dimethoxy-2,3,4,5-tetrahydro-1 H-3-benzazepin-2-one. Ivabradine has the following structural formula:

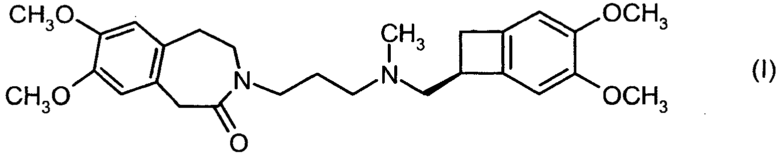

Synthesis routes for the preparation of ivabradine and its use for preventing and treating various clinical conditions of myocardial ischaemia, supraventricular arrhythmias and coronary arteriosclerotic episodes are reported to be disclosed in EP 534 859.

Ivabradine is an active substance reported to have a bradycardic effect for the treatment of stable angina pectoris, in particular in patients for whom beta-blockers are contraindicated or intolerance of beta-blockers is present. Ivabradine is reported to selectively inhibit the I_{f}-ion current which, as an intrinsic pacemaker in the heart, controls the spontaneous diastolic depolarisation in the sinoatrial node and thus regulates the heart rate. Under physiological conditions, ivabradine, the S-enatiomer of a racemate, is reported to have a very good water solubility (> 10 mg/ml).

The prior art apparently discloses administration forms of ivabradine which release the active substance substantially without a time delay. The administration form Procoralan^{®} (Servier) releases ivabradine rapidly and almost completely after oral intake. WO 2003-061662 apparently discloses an ivabradine-containing, orally dispersible tablet, which releases the active substance very rapidly in the mouth.

However, administration of ivabradine with immediate release has disadvantages since it can lead to very high plasma concentrations too quickly in certain patients.

In general, non-retarded oral or parenteral administration forms do not result in the rapid build-up of plasma levels after a single administration. After the end of the resorption, however, there is a more or less rapid drop in the plasma concentration of the active substance, depending on the magnitude of the elimination constant. In the case of substances having a fast elimination constant, this leads to considerable variations in the plasma level on repeated administration and may cause a temporary decline in the effect and, if a higher dose is required, also the possible occurrence of side-effects.

An administration form with modified release can avoid these peak values of the plasma concentration and ensure a uniform concentration of the active substance in the blood. Sustained-release dosage forms have the advantage that they can avoid subtherapeutic and toxic plasma or tissue concentrations and instead maintain suitable plasma or tissue concentrations over a longer period. An advantage of an administration form with modified release is that frequently the total dose can be reduced while achieving a comparable effect or the duration of action is prolonged. Furthermore, the patient compliance is generally improved and the frequency of intake can be decreased.

The modified release of ivabradine would thus make it possible to provide an improved therapeutic index. However, it is necessary for this purpose to ensure an active substance release which is modified over time.

One possibility for achieving the modified release of the active substance is based on the embedding of the active substance in a matrix which is formed from a polymer. There is a multiplicity of known polymers which can be used for forming such a matrix. However, in the case of ivabradine, the preparation of pharmaceutical compositions with modified release is complicated by specific preparation problems. In particular, it is found that the stability of the active substance in the course of the preparation process is a substantial factor.

Moreover, in the case of highly water-soluble active substances, such as ivabradine salts, the preparation of drugs with modified release is not easy and difficulties frequently occur, such as, for example,
- a complicated preparation process,
- the stability of the active substances and the excipients,
- the correct establishment of the desired release profile,
- the reproducibility of the batches.

These and further problems are discussed in WO 2002/051387.

WO 2002/051387 claims to disclose a process for the preparation of solid pharmaceutical formulations for the controlled release of ivabradine through the use of specific thermoforming techniques. Exclusively polymethacrylates are used as excipients, it not being permitted for any plasticiser or agent which modulates the release of the active substance to be present. However, the thermoforming techniques described are extremely complicated and require high material consumption and necessitate very exact temperature control. The most complicated disadvantage is the necessary increase in temperature which, depending on the salt or polymorph of ivabradine used, leads to more or less pronounced decomposition thereof. In this publication no composition is disclosed comprising a combination of water-soluble and water-insoluble excipients.

Ivabradine may be present in various polymorphic forms, which additionally complicates the development of ivabradine formulations. Various polymorphic forms of ivabradine hydrochloride are reported to be described in the prior art. WO 2005/110993 A1 apparently discloses polymorph alpha, WO 2006/092493 A1 apparently discloses polymorph beta and WO 2006/092491 A1 apparently discloses polymorph beta d (dehydrated). Furthermore, polymorph gamma, polymorph gamma d, polymorph delta and polymorph delta d are apparently disclosed in the prior art. In addition, WO2008/065681 apparently reports the so-called Form I of Ivabradine HCl. WO 2008/146308 A2 apparently discloses amorphous ivabradine.

Various salts of ivabradine are apparently also disclosed in the prior art. WO 2008/146308 A2 apparently discloses ivabradine oxalate and WO 2009/124940 A1 apparently discloses ivabradine hydrobromide.

CN 101028518 A apparently discloses formulations containing nitrates and ivabradine.

In the case of the salts and polymorphs of ivabradine, in particular the polymorphs of the hydrochloride, there is the problem that these salt forms are not sufficiently stable under all conditions. This in turn can lead to problems in the processing and storage and to undesired reactions with the excipients used in the preparation of the pharmaceutical composition.

The different solubility profiles of the polymorphic forms lead to an undesirable nonuniform uptake of the active substance in the patient. It was therefore also an object of the present invention to provide stable forms of ivabradine which can be processed to give an administration form which permits as uniform an uptake as possible in the patient. Both interindividual and intraindividual deviations should be substantially avoided.

There is therefore a need for ivabradine-containing formulations with modified release, which can provide both a rapid onset of action and a long-lasting release. There is in particular a need for defined control of the release rate and of the bioavailability of the active substance. In addition, it is desirable to improve the chemical stability of the active substance and the stability of the polymorphs, in particular to hydrolytic degradation and under thermal load. A further problem consists in the improvement of the processability of ivabradine to pharmaceutical compositions, in particular those with modified release, in the reduction of the hygroscopicity of the composition and the improvement of the storage stability of corresponding compositions. Finally, it is intended to provide novel salt forms of ivabradine which can be particularly advantageously stabilized in a composition with modified release.

It has now surprisingly been found that the abovementioned objects can be achieved by the combination of the active substance ivabradine in the form of its adipate salt (a) with a water-soluble (b) and a water-insoluble excipient (c) in a pharmaceutical composition with modified release. By embedding the active substance (a) in a mixture of the water-insoluble and the water-soluble excipient, surprisingly not only can the active substance advantageously be released in a modified manner but it is also stabilized in its polymorphic form as well as chemically. Corresponding compositions show little hygroscopicity and outstanding modified release. Corresponding mixtures can be further processed in an excellent manner to give pharmaceutical compositions, in particular tablets.

Furthermore, it was found that some salts of ivabradine are particularly suitable for the compositions according to the invention with modified release and can be used in the drugs according to the invention with modified release. These salts are novel compounds, and the invention also relates to these salts. The novel salts are advantageously stabilized in the novel formulations.

The present invention therefore relates to a pharmaceutical composition for modified release, which comprises ivabradine adipate and a combination of a water-insoluble and a water-soluble excipient. The active substance ivabradine is present as adipate, i.e. in the form of its salt with adipic acid. In the context of this invention, ivabradine adipate can be used both in amorphous form and in crystalline form. The composition is intended for oral administration. Preferably the pharmaceutical composition is a stable pharmaceutical composition.

Here, "active substance" or "ivabradine" is understood as meaning ivabradine in the form of the adipate salt, i.e. the salt of ivabradine with adipic acid.

The present invention also discloses the following salts of: L-aspartate, D-aspartate, sorbate, acinotate, gluconate, glucoheptonate, glucuronate, hippurate, 8-chlorotheophyllinate, xinafoate, malate, sulphamate, oxalate form I, oxalate form II, oxalate form III and salts of ivabradine with ethanesulphonic acid, ethane-1,2-disulphonic acid, naphthalenesulphonic acid, naphthalenecarboxylic acid, embonic acid, mandelic acid, lactobionic acid or adipic acid. The salts of ivabradine can be obtained according to processes for the preparation of ivabradine salts reported to be known to the person skilled in the art, e.g. by reacting the free base of ivabradine with the corresponding acid or by the presence of the corresponding acid in the synthesis of ivabradine, as reported to be described, for example, in US 2005/0228177 A1.

Ivabradine adipate can be obtained by adding adipic acid, e.g. about one equivalent, in a suitable solvent, such as ethanol, to a solution of ivabradine in a suitable solvent, such as dichlormethane. Crystalline ivabradine adipate product can be obtained by removal of the solvent, e.g. under vacuum at about 40°C. Crystalline ivabradine adipate can also be obtained by adding a solution of adipic acid in water to a solution of ivabradine in ethanol, and removal of the solvent.

The DSC thermogramm of ivabradine adipate shows a peak at about 115°C. The melting point is in the range of about 113°C to about 117°C.

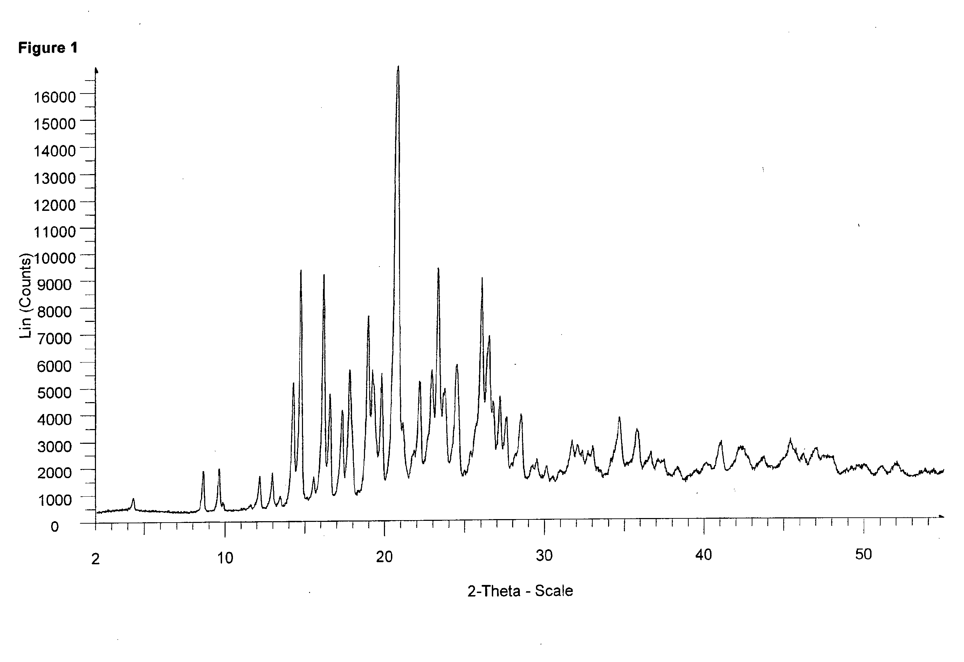

Ivabradine adipate is characterized by an XRD pattern having a characteristic peak at 20.6 ± 0.2 degrees 2-theta, in particular having characteristic peaks at 14.6 ± 0.2, 16.0 ± 0.2, 18.8 ± 0.2, 20.6 ± 0.2, 23.2 ± 0.2, 24.3 ± 0.2, 25.9 ± 0.2 and 26.3 ± 0.2 degrees 2-theta and preferably further at 8.6 ± 0.2, 9.6 ± 0.2, 12.1 ± 0.2 and 12.9 ± 0.2 degrees 2-theta. The XRD pattern of ivabradine adipate is shown in Figure 1.

Ivabradine oxalate I is characterized by an XRD pattern having a characteristic peaks at 17.7 ± 0.2 and 23.0 ± 0.2 degrees 2-theta, in particular having characteristic peaks at 16.7 ± 0.2, 17.1 ± 0.2, 17.7 ± 0.2, 20.9 ± 0.2, 23.0 ± 0.2, 23.6 ± 0.2, 24.4 ± 0.2 and 27.5 ± 0.2 degrees 2-theta or at 16.7 ± 0.2, 17.7 ± 0.2, 19.0 ± 0.2, 20.9 ± 0.2, 23.6 ± 0.2, 24.4 ± 0.2 and 27.5 ± 0.2 degrees 2-theta.

Ivabradine oxalate II is characterized by an XRD pattern having a characteristic peaks at 17.3 ± 0.2 degrees 2-theta, in particular having characteristic peaks at 13.5 ± 0.2, 17.3 ± 0.2, 18.2 ± 0.2, 19.1 ± 0.2, 21.1 ± 0.2, 23.3 ± 0.2, 25.6 ± 0.2 and 27.3 ± 0.2 degrees 2-theta or at 13.5 ± 0.2, 17.3 ± 0.2, 21.1 ± 0.2, 23.3 ± 0.2, 25.6 ± 0.2 and 27.3 ± 0.2 degrees 2-theta.

Ivabradine oxalate III is characterized by an XRD pattern having characteristic peaks at 20.9 ± 0.2 and 23.1 ± 0.2 degrees 2-theta, in particular having characteristic peaks at 16.9 ± 0.2, 17.5 ± 0.2, 18.8 ± 0.2, 19.9 ± 0.2, 20.9 ± 0.2, 23.1 ± 0.2, 25.5 ± 0.2 and 32.3 ± 0.2 degrees 2-theta or at 16.9 ± 0.2, 17.5 ± 0.2, 20.9 ± 0.2, 23.1 ± 0.2, and 32.3 ± 0.2 degrees 2-theta.

Corresponding to the preparation of ivabradine adipate, ivabradine-(S)-mandalate, ivabradine-1-hydroxy-2-naphthanoate, ivabradine-L-aspartate, ivabradine-D-aspartate, ivabradine ethanesulphonate and ivabradine-L-malate can be prepared by reacting ivabradine free base in a suitable solvent, e.g. acetronitrile or dichloromethane, with the corresponding acid.

Furthermore, amorphous ivabradine, in the form of the above described adipate salt is preferably used in the pharmaceutical compositions with modified release of the present invention.

The invention also relates to the use of a combination of water-soluble and water-insoluble excipients for the preparation of an ivabradine adipate-containing oral administration form, preferably of a tablet with modified release.

Preferably, the pharmaceutical compositions of the present invention contain about 1 - 80 % by weight, more preferably about 2 - 60 % by weight, in particular about 2 - 40 % by weight, e.g. about 2 - 20 % by weight, of ivabradine adipate, based on the weight of free base in the composition.

All pharmaceutically acceptable excipients having sufficient water solubility can be used in the pharmaceutical compositions with modified release as suitable water-soluble excipients (component (b)), provided that they do not counteract the desired modified release. Such excipients are known to the person skilled in the art. The solubility in water of the water-soluble excipients is greater than or equal to 33 mg/ml, measured at 25°C, in particular greater than or equal to 100 mg/ml, measured at 25°C. Suitable water-soluble excipients are cellulose derivatives, such as, for example, hydroxypropylmethylcellulose, hydroxyethylcellulose, hydroxypropylcellulose, sodium carboxymethylcellulose (sodium-CMC), cellulose acetate phthalate and hydroxypropylmethylcellulose phthalate, or polyvinylpyrrolidone (PVP), copolymers of PVP and vinyl acetate and polyvinyl alcohol, polyethylene glycol (PEG) and xylitol, sorbitol or other sugar alcohols, or sodium chloride. Preferably, the water-soluble excipients consist chemically of more than one monomer, i.e. they are polymers, preferably the cellulose derivatives and/or polyvinylpyrrolidone (PVP) or its copolymers and PEG. In the context of this invention, the water solubility is determined according to EU-Directive RL67-548-EEC, Annex V Section A6, at pH = 7.

All pharmaceutically acceptable excipients having insignificant water solubility at 25°C can be used in the pharmaceutical compositions with modified release as suitable water-insoluble excipients (component (c)), provided that they do not counteract the desired modified release. Such excipients are known to the person skilled in the art. The solubility in water of the water-insoluble excipients is less than 33 mg/ml, measured at 25°C, in particular 10 mg/ml or less, measured at 25°C. Particularly preferably, the water solubility of these excipients is 1 mg/ml or less, likewise at 25°C. Suitable water-insoluble excipients are water-insoluble cellulose derivatives; such as, for example, ethylcellulose, ethylhydroxyethylcellulose, microcrystalline cellulose or cellulose powder, polyvinyl acetate, starches, stearic acid, waxes, glyceryl palmitate stearate, but also ion exchange resins, such as polacrilin potassium, sodium polystyrenesulphonate and/or colestyramine resinate. Water solubility is determined according to EU Directive RL67-548-EEC, Annex V Section A6 at pH = 7.

Water-soluble and/or water-insoluble cellulose derivatives having a weight average molecular weight of, preferably, in the range from 5000 to 1 million g/mol, particularly preferably in the range from 10000 to 100000 g/mol, are preferably used as excipients.

The water-soluble and/or water-insoluble excipients which are used in combination in the pharmaceutical compositions with modified release according to the present invention are typically each present in an amount of about 2 - 80 % by weight, preferably about 5 - 60 % by weight, in particular about 8 - 30 % by weight, based on the total weight of the composition. The sum of the proportions of the ingredients of the composition is in each case 100% by weight.

The finished administration forms may be oral administration forms, for example tablets, capsules, sachets or powders. Preferably, the pharmaceutical composition according to the invention is present in the form of a tablet. The preparation of tablets by means of granulation or direct compression of the ingredients is known in the prior art. Tablets according to the invention are preferably prepared by direct compression.

The pharmaceutical composition may contain one or more pharmaceutically acceptable excipients which may be water-soluble or water-insoluble, provided that they do not counteract the desired modified release. For example, fillers, glidants, flow regulators and/or release agents may be mentioned by way of example ("Lexikon der Hilfsstoffe für Pharmazie, Kosmetik und angrenzende Gebiete [Lexikon of excipients for pharmacy, cosmetics and ancillary areas]", edited by H.P Fiedler, 4th edition, and "Handbook of Pharmaceutical Excipients", 3rd edition, edited by Arthur H. Kibbe, American Pharmaceutical Association, Washington, USA und Pharmaceutical Press, London).

Filler: The pharmaceutical composition may contain one or more fillers. In general, a filler is a substance which increases the bulk volume of the mixture and hence the size of the resulting dosage form. Preferred examples of fillers are lactose and calcium hydrogen phosphate. The filler may have a proportion of about 0 % to 99 % by weight, preferably between about 40 % and 96 % by weight, of the total weight of the composition.

Glidant: The function of the glidants is to ensure that the tablet compression and the ejection take place without major friction between the solids and the walls. The glidant is preferably an alkaline earth metal stearate, such as magnesium stearate, or a fatty acid, such as stearic acid. The glidant is usually present in an amount of about 0 % - 2 % by weight, preferably between about 0.5 % and 1.5 % by weight, of the total weight of the pharmaceutical composition.

Flow regulator/release agent: For example, colloidal silica or magnesium stearate can be used as a flow regulator and/or release agent. The flow regulator/release agent is preferably present in an amount of about 0 % - 8 % by weight, more preferably in an amount between about 0.1 % and 3 % by weight, of the total weight of the composition.

Methods for controlling the release of the active substance, by means of which the release rate, the time or the site (e.g. stomach or intestine) of the release of the active substance or of the active substances can be adjusted as intended, are generally known in the art.

In the pharmaceutical compositions according to the present invention, the release is preferably modified by the formation of a suitable hydrophilic matrix. Preferably, a hydrophilic matrix which releases the active substance in a modified manner is formed by the combination of the water-insoluble and of the water-soluble excipient according to the invention.

Furthermore preferred according to the invention is a drug formulation having a core which comprises the active substance and the combination, according to the invention, of a water-insoluble and a water-soluble excipient and which is provided with a coating which modifies, i.e. in particular retards, the release. The core without the coating preferably releases the active substance rapidly. The release-controlling coatings are known in the prior art, for example those which are based on polymethacrylates, such as coating materials of the Eudragit series.

Further known methods for modifying the release of an active substance can likewise be used according to the invention, for example retardation by embedding in fat, the use of ion exchange resins, the use of epoxy resin .beads, retardation by formation of a solid solution or by complex formation with the active substance, and osmotic systems.

In a preferred embodiment, the pharmaceutical composition is a film-coated tablet. In a preferred embodiment, the constituents (a), (b) and (c) and optionally the further excipients described above therefore form a tablet core, the tablet core preferably being provided with a coating (= component (d)).

In this invention, three different coatings (d) are in general preferred:
(d1) coatings without any influence on the active substance release;
(d2) enteric coatings; and
(d3) sustained-release coatings.

Coatings without any influence on the active substance release are usually water-soluble (they preferably have a water solubility of more than 250 mg/ml). Enteric coatings have a pH-dependent solubility. Sustained-release coatings are usually water-insoluble (they preferably have a water solubility of less than 10 mg/ml).

Macromolecular substances, for example modified celluloses, polymethacrylates, polyvinylpyrrolidone, polyvinyl acetate phthalate, zein and/or schellac or natural gum, such as, for example, carrageenan, are usually used for the coating (d).

Preferred examples of coating polymers which have no influence on the active substance release (d1) are water-soluble polymers, such as, for example, low-viscosity hydroxypropylmethylcellulose (HPMC), hydroxypropylcellulose (HPC), hydroxyethylcellulose (HEC), polvinylpyrrolidone (PVP) and mixtures thereof. Said polymers should usually have a weight average molecular weight of 10000 to 150000 g/mol. HPMC is preferably used, in particular HPMC having a weight average molecular weight of 10000 to 150000 g/mol and/or an average degree of substitution of -OCH3 groups of 1.2 to 2.0.

Examples of enteric coatings (d2) are those which are based on cellulose acetate phthalate (CAP), hydroxypropylmethylcellulose phthalate and polyvinyl acetate phathalate (PVAP).

Examples of sustained-release coatings (d3) are those which are based on ethyl cellulose (EC, commercially available, for example, as Surelease®) and polymethacrylates (commercially available, for example, as Eudragit® RL or RS and L/S).

The coating (d) may be free of active substance. However, it is also possible for the coating (d) to contain active substance (a).

In one preferred embodiment in the pharmaceutical compositions according to the invention the active substance is not present as a mixture with a polyacrylate or a polymethacrylate.

In addition to the active substance ivabradine adipate (or a solvate, hydrate or polymorph thereof), the pharmaceutical composition according to the invention may also contain active substances differing from ivabradine adipate; preferably, the pharmaceutical composition according to the invention contains exclusively ivabradine adipate as the only active substance (or solvate, hydrate or polymorph thereof) and no further active substance.

The pharmaceutical composition according to the present invention and which is preferably present in the form of a tablet which contains the constituents (a), (b) and (c) and optionally (d), is a composition with modified release. In the context of this invention, the term modified release is understood as meaning delayed release, prolonged release, sustained release or extended release. According to the invention, the term "modified release" is preferably used in the sense of "sustained release". The above terms are usual in the technical area and reference may be made, for example, to the textbook Voit, "Pharmazeutische Technologie", 9^{th} edition (2000).

Here, modified release is preferably understood as meaning that the composition, preferably in the form of pellets or tablets, releases the active substance after a time delay, i.e. has a release profile, so that the total active substance is not released in less than 2 hours, preferably not released in less than 4 hours. In a preferred embodiment of the present invention, the pharmaceutical composition according to the invention has a release profile of the active substance wherein 1-35%, preferably 5-30% of the active substance are released after one hour, 5-40%, preferably 10-35%, after two hours, 10-50%, preferably 15-40%, after six hours and 15-100%, preferably 50-80%, after 16 hours. More preferred compositions are those having a release profile wherein 1-35%, preferably 5-30% of the active substance are released after one hour, 5-50%, preferably 15-45%, after two hours, 10-80%, preferably 25-70%, after six hours and 20-100%, preferably 50-97%, after 16 hours.

The pharmaceutical compositions according to the invention are usually distinguished by release and absorption which lead to advantageous values of cₘₐₓ (maximum plasma level).

In a preferred embodiment, the oral administration of the formulation according to the invention to a human as a patient leads to a plasma level profile which is distinguished by a cₘₐₓ, based on a single daily intake of 10 mg of the active substance ivabradine, in the steady state, of about 5 to 40 ng/ml, preferably 10 to 30 ng/ml.

The abovementioned values for the plasma level are preferably mean values, obtained by investigations of blood samples of a group of 10 test subjects (having an average body weight of 70 kg), the corresponding blood samples having been taken 0, 1, 2, 3, 4, 6, 8, 12, 24 and 48 hours after oral administration of the composition according to the invention in the steady state. The determination of the plasma level values can preferably be carried out by suitable HPLC-MSMS methods.

In the context of the present invention, "release profile" is understood as meaning the variation of the amount of the active substance which is present in solution, based on the total amount of the active substance, as a function of time. According to the invention, the release profile is determined by dissolving a solid administration form of the pharmaceutical composition of the present invention in a USP apparatus I (basket apparatus) in 500 ml of USP buffer (phosphate buffer) at pH 6.8, 37°C and a stirrer speed of 50 rpm and measuring the amount of dissolved active substance over a period, e.g. 24 hours, at the desired times. The content of active substance released can be determined in a known manner, for example by absorption of light of a certain wavelength or by means of HPLC or another suitable detection method. Otherwise, the test conditions for determining the release profile correspond to the methods of the US Pharmacopoeia.

The pharmaceutical compositions according to the invention are preferably present as tablets which contain ivabradine adipate in an amount of preferably 1 mg to 25 mg, more preferably of 3 mg to 20 mg, in particular of 10 mg to 15 mg, based on ivabradine free base. The invention therefore relates in particular to tablets containing 5 mg, 10 mg and 15 mg of ivabradine adipate, based on ivabradine free base.

The pharmaceutical compositions according to the invention are preferably administered once daily, in particular in the form of a tablet.

Finally, the tablets according to the invention usually have a content uniformity of 95 to 105 %, preferably of 98 to 102 %, in particular of 99 to 101 %, of the average content (this means that all tablets have an active substance content between 95 and 105 %, preferably between 98 to 102 %, in particular between 99 and 101 %, of the average active substance content.) The "content uniformity" is determined according to Ph. Eur. 6.0, Section 2.9.6.

The present invention furthermore relates to a process for the preparation of a pharmaceutical composition according to the present invention as described above comprising the steps of mixing ivabradine adipate with the water-insoluble and the water-soluble excipient, optionally with further pharmaceutically acceptable excipients, optionally isolating the composition obtained and optionally converting the composition into a solid pharmaceutical dosage form, preferably a tablet, optionally followed by coating of the dosage form. The production of tablets, for example by means of granulation or direct compression of the ingredients, is known in the art. The tablet according to the invention is preferably produced by direct compression.

Methods for the preparation of drugs with modified release are known to the person skilled in the art, and reference may be made, for example, to the textbook Bauer, Frömming und Führer, "Lehrbuch der Pharmazeutischen Technologie", 6th edition 1999. According to the invention, the methods described in this textbook can be used analogously.

The present invention furthermore relates to the use of a combination of a water-insoluble and a water-soluble excipient, in particular those mentioned above as being preferred, in a pharmaceutical composition with modified release, containing ivabradine adipate, in particular for delaying the release of the active substance ivabradine adipate.

The present invention furthermore relates to the use of a combination of a water-soluble and a water-insoluble excipient as a mixture with the active substance ivabradine adipate in a pharmaceutical composition with modified release for stabilizing the active substance.

Attached Figure 1 shows an XRD pattern of ivabradine adipate.

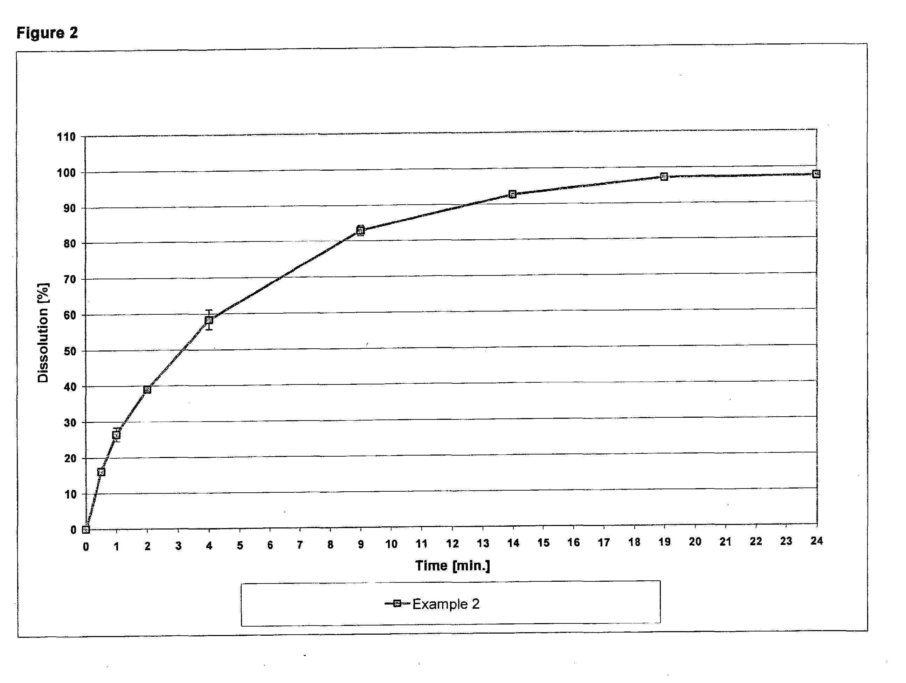

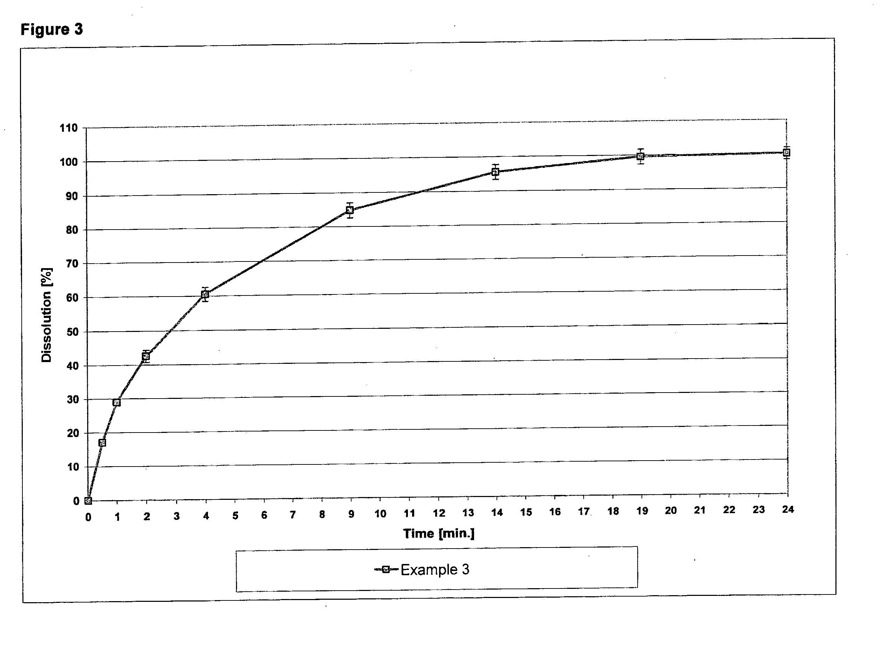

Figures 2 and 3 show dissolution profiles of the compositions of examples 2 and 3, respectively.

**XRD** samples were analysed on a Bruker-AXS D8 Advance powder X-Ray diffractometer. The measurement conditions were as follows :

| | | |
|---|---|---|
| Measurement in Bragg-Brentano-Geometry on vertical goniometer (reflection, theta/theta, | | |
| | 435 mm measurement circle diameter) | |
| | with sample rotation (30 rpm) on 9 position sample stage | |
| Radiation: | Cu Kα1(1.5406Å), Tube (Siemens FLCu2K), power 40kV/40mA | |
| Detector: | position sensitive detector VANTEC-1 | |
| | 3° capture angle (2theta), | |
| | Anti scatter slit | 6.17 mm |
| | Detector slit | 10.39 mm |
| | 4° soller slit, | |
| | primary beam stop (<2° 2theta) | |
| Monochromator: | None | |
| Second β filter: | Ni filter 0.1 mm (0.5%) | |
| Start angle: | 2° | |
| End Angle: | 55° | |
| Measurement time: | 11 min | |
| Step: | 0.016° 2Theta | |
| Software: | EVA (Bruker-AXS, Karlsruhe). | |

DSC samples were analyzed on a Mettler Toledo Model DSC 822.
The measurement conditions were as follows: Heating range for the samples 30 to 300°C; heating rate = 10°C / min. Purge gas = nitrogen 50 ml/min; 40 micron aluminum crucible.

The present invention will now be explained in more detail with reference to the following examples, which are not intended as being limiting.

### Example 1: Retardation by coating

A tablet of the following composition was produced:

| *Core:* | |
|---|---|
| Ivabradine adipate | 6.56 mg |
| Avicel PH101 | 45.97 mg |
| Sorbitol | 45.97 mg |
| Aerosil | 1.00 mg |
| Magnesium stearate | 0.50 mg |
| | **100.0 mg** |

| *Coating:* | |
|---|---|
| Eudragit RS 30D | 6.35 mg |

The cores were produced by mixing the ingredients ivabradine, Avicel, sorbitol and Aerosil and subsequently adding the magnesium stearate. The cores were then coated with an aqueous suspension containing Eudragit RS 30D.

In this formulation, sorbitol is a water-soluble excipient and Avicel PH101 is a water-insoluble excipient.

### Example 2: Retardation by formation of a hydrophilic matrix

| | |
|---|---|
| Ivabradine adipate | 6.51 mg |
| Hypromellose | 40.91 mg |
| Calciumhydrogenphosphate | 15.00 mg |
| Avicel PH 101 | 35.00 mg |
| Aerosil | 1.58 mg |
| Magnesium stearate | 1.00 mg |
| | **100.00 mg** |

The ingredients were mixed in a mixer and then pressed to give tablets.

In this formulation, in particular the HPMC (Methocel K15M) is a water-soluble excipient and the Avicel PH101 is a water-insoluble excipient

The dissolution profile (conditions: 500 mL 50 mM Phosphate buffer pH 6.8 - 37°C - 50 rpm baskets (USP app. I)) of the tablet according to Example 2 is shown in Fig. 2.

### Example 3: Retardation by formation of a hydrophilic matrix (not according to the invention)

| | |
|---|---|
| Ivabradine HCl | 5.42 mg |
| Hypromellose | 42.00 mg |
| Calciumhydrogenphosphate | 15.00 mg |
| Avicel PH 101 | 35.00 mg |
| Aerosil | 1.58 mg |
| Magnesium stearate | 1.00 mg |
| | **100.00 mg** |

The ingredients were mixed in a mixer and then pressed to give tablets.

In this formulation, in particular the HPMC (Methocel K15M) is a water-soluble excipient and the Avicel PH101 is a water-insoluble excipient

The dissolution profile (Conditions: 500 mL 50 mM Phosphate buffer pH 6.8 - 37°C - 50 rpm baskets (USP app. I)) of the tablet according to Example 3 is shown in Fig. 3.

### Example 4: Release profiles

For the dosage forms according to example 1 and example 2, release profiles were measured. The release profile was determined by dissolving a solid administration form of the pharmaceutical composition of the present invention in a USP apparatus I (basket apparatus) in 500 ml of a USP buffer (phosphate buffer) and pH 6.8, 37°C and a stirrer speed of 50 rpm and measuring the amount of dissolved active substance at the stated times. The content of active substance released was determined by means of HPLC. An active substance release according to the following table was found:

| **Time (h)** | **Release (%)** |
|---|---|
| 1 | 1-35 |
| 2 | 5-50 |
| 6 | 10-80 |
| 16 | 20-100 |

### Example 5a: Ivabradine adipate

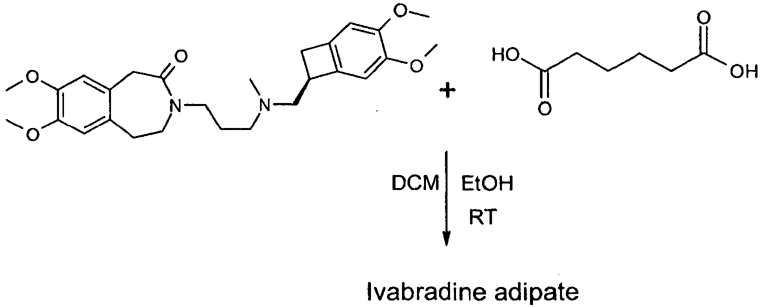

A solution of 0.095 g (0.649 mmol, 1.0 eq) adipic acid in 1.5 ml ethanol was added to a solution of 0.304 g (0.649 mmol) ivabradine freebase in 2 ml dichloromethane at room temperature. After 16 h the solvent was evaporated in vacuo and the residue was triturated with 4 ml dry diethyl ether. The solid was filtered and washed with 2 ml dry diethyl ether, dried at 40 °C under vacuum for 4 h to give 0.336 g (84%, 0.546 mmol) of ivabradine adipate as white solid.
**IR**: 2999.8, 2943.9, 2913, 2861.4, 2833.4, 1697.3, 1643.4, 1518.3, 1486.7, 1466.7, 1370.9, 1306.7, 1245.1, 1224.5, 1210.7, 1105.4, 1060.8, 862.8, 828, 771.2, 638.2, 499.4, 458.
**DSC**: 115.6 °C (-121.40 J/g). **M.P**.: 113-117 °C
**HPLC**: 98.55 %

The XRD pattern of ivabradine adipate is shown in Figure 1.

### Example 5b: Ivabradine adipate

A solution of 0.064 g (0.437 mmol, 1.0 eq) adipic acid in 1.5 ml water was added to a solution of 0.205 g (0.437 mmol) ivabradine freebase in 2 ml ethanol. After stirring at room temperature for 2 h the solvent was evaporated in vacuo and the residue was triturated with 6 ml dry diethyl ether, followed by stirring for 30 min. The solid was filtered, washed with 2 ml dry diethyl ether, and dried at 45 °C under vacuum for 4 h to give 0.237 g (88%, 0.385 mmol) of ivabradine adipate as white solid.

**IR**: 2999.1, 2944, 2913, 2862.2, 2833.6, 1704, 1644.6, 1518.5, 1486.7, 1466.7, 1371.1, 1306.2, 1245.7, 1224.3, 1210.7, 1105.7, 1060.8, 862.7, 828.3, 771.2, 638.2, 500.1, 458.

DSC shows a sharp endothermic peak at 113.2°C confirming the crystalline nature of the salt.

### Example 6: Ivabradine-(S)-mandelate (not according to the invention)

A solution of 0.032 g (0.213 mmol, 1.0 eq) (S)-(+)-mandelic acid in 0.5 ml acetonitrile was added to 0.100 g (0.213 mmol, 1.0 eq) ivabradine freebase in 0.5 ml dichloromethane at room temperature. Reaction was stirred at room temperature for 2 h and subsequently evaporated to dryness. The residue was triturated with 4 ml dry diethyl ether and then with 4 ml n-hexane, solvent was decanted out and the solid was dried at 40 °C for 2 h to give 0.106 g (80 %, 0.171 mmol) of ivabradine-(s)-mandelate.
DSC and XRD show amorphous nature of the salt.

### Example 7: Ivabradine-1-hydroxy-2-naphthanoate (not according to the invention)

A solution of 0.044 g (0.235 mmol, 1.0 eq) 1-hydroxy-2-naphthoic acid in 0.7 ml ethanol was added to a solution of 0.110 g (0.235 mmol) ivabradine freebase in 0.5 ml dichloromethane at room temperature. After 1 h, the solvent was evaporated in vacuo to give a foamy residue which was triturated with 4 ml dry diethyl ether. The supernatant solvent was decanted out. Trituration was repeated with dry diethyl ether, the supernatant solution was decanted out and the solid was dried at 40 °C under vacuum for 2.5 h to give 0.138 g (90%, 0.210 mmol) of light brown solid.
DSC and XRD confirm the amorphous nature of the salt.

### Example 8: Ivabradine-L-aspartate (not according to the invention)

A solution of 0.030 g (0.224 mmol, 1.0 eq) L-Aspartic acid was prepared in 2 ml water by refluxing. A solution of 0.105 g (0.224 mmol, 1.0 eq) ivabradine freebase in 1 ml acetonitrile was added at reflux temperature and refluxed for 15 min. Reaction mixture was stirred at room temperature for overnight. The solvents were evaporated in vacuo and the residue was triturated with 4 ml dry diethyl ether. Solid obtained was dried at 40 °C under vacuum for 2 h to give 0.116 g (86%, 0.193 mmol) of the salt.
DSC and IR confirm amorphous nature of the salt.

### Example 9: Ivabradine-D-aspartate (not according to the invention)

A solution of 0.029 g (0.218 mmol, 1.0 eq) D-Aspartic acid was prepared in 1.5 ml water by refluxing. A solution of 0.102 g (0.218 mmol, 1.0 eq) ivabradine freebase in 1 ml acetonitrile was added at reflux temperature and refluxed for 15 min. Reaction mixture was stirred at room temperature for overnight. The solvents were evaporated in vacuo and the residue was triturated with 4 ml dry diethyl ether followed by 4-5 ml n-hexane and solvents decanted out. Trituration was repeated once again and solvent decanted out. The solid was dried at 40 °C under vacuum for 2 h to give 0.100 g (76%, 0.166 mmol) of the salt.
DSC showed the amorphous nature of the salt.

### Example 10: Ivabradine ethanesulphonate (not according to the invention)

To a solution of 0.102 g ivabradine freebase (0.218 mmol, 1.0 eq) in 0.5 ml dichloromethane, 0.018 g (0.218 mmol, 1.0 eq) ethane sulphonic acid was added at room temperature and the mixture was stirred for overnight. The reaction mixture was evaporated to dryness and triturated with 2 ml dry diethyl ether, followed by 2 ml n-hexane. The resulting foamy solid was evaporated to dryness at 40 °C under vacuum for 2h to give 0.101 g (80%, 0.174 mmol) of white solid.
DSC and XRD confirm the amorphous nature of the salt.

### Example 11: Ivabradine-L-malate (not according to the invention)

A solution of 0.086 g (0.64 mmol) L-malic acid in 1.5 ml acetonitrile was added to a solution of 0.3 g (0.64 mmol) ivabradine freebase in 1 ml acetonitrile at room temperature and was stirred for overnight. The solution was evaporated to dryness and the residue was triturated with 4 ml dry diethyl ether, followed by trituration with 4 ml n-hexane. The solvent was evaporated and the solid was dried at 40°C under vacuum for 2 h to yield 0.302 g of solid.
HPLC = 98 %
DSC and XRD confirm the amorphous nature of the salt.

### Example 12a: Ivabradine oxalate I (not according to the invention)

To the solution of 100 mg ivabradine (0.213 mmol, 1.0 eq) in 0.5 ml acetone was added a solution of 19 mg oxalic acid (0.213 mmol, 1.0 eq) in 0.5 ml acetone. Within minutes a solid separated and additional 2 ml acetone were added to enable stirring. The solid was isolated by filtration and washed with 2 x 2 ml acetone. It was dried for 3 h at 45 °C to give 96 mg (81%, 0.172 mmol) of ivabradine oxalate.

| | |
|---|---|
| **IR** | : 2937.8, 2834.8, 1714.5, 1642.3, 1519.2, 1485.8, 1467.0, 1322.2, 1304.9, 1280.3, 1248.0, 1210.9, 1185.3, 1110.9, 1070.7, 1030.8, 832.4, 699.4, 472.2 |
| **MP** | : 102.3-115.6 °C (Not sharp, melted over a range) |
| **DSC** | : 109.3 °C (-65.2 J/g), 152.3 °C (-0.5 J/g), 158.6 °C (-4.5 J/g) |

Ivabradine oxalate I is characterized by an XRD pattern having peaks at 16.7 ± 0.2, 17.7 ± 0.2, 19.0 ± 0.2, 20.9 ± 0.2, 23.0 ± 0.2, 24.4 ± 0.2 and 27.5 ± 0.2 degrees 2-theta.

### Example 12b: Ivabradine oxalate II (not according to the invention)

To the solution of 320 mg ivabradine (0.683 mmol, 1.0 eq) in 4 ml DCM was added a solution of 61.5 mg oxalic acid (0.683 mmol, 1.0 eq) in 1 ml ethanol and the mixture was stirred at room temperature for 2 h. The solvent was evaporated in vacuo to give a solid which was dried for 4 h at 40 °C, yielding 340 mg (89%, 0.609 mmol) of ivabradine oxalate.

| | |
|---|---|
| **IR** | : 2943.5, 2914.5, 1730.3, 1651.7, 1521, 1484.4, 1466.0, 1304.6, 1248, 1221.8, 1210.1, 1105, 1074.5, 706.5, 486.4 |
| **DSC** | : 145.4 °C (-53.0 J/g) |
| **HPLC** | : 95.4% |

Ivabradine oxalate II is characterized by an XRD pattern having peaks at 13.5 ± 0.2, 17.3 ± 0.2, 21.1 ± 0.2, 23.3 ± 0.2, 25.6 ± 0.2 and 27.3 ± 0.2 degrees 2-theta.

### Example 12c: Ivabradine oxalate III (not according to the invention)

To the solution of 225 mg ivabradine (0.480 mmol, 1.0 eq) in 2 ml acetone was added a solution of 43 mg oxalic acid (0.480 mmol, 1.0 eq) in 1 ml acetone at room temperature. Within minutes a solid separated. After addition of 3 ml more acetone the mixture was stirred at room temperature for 1 h. The solid was then isolated by filtration and washed with 4 ml acetone. It was dried at first for 4 h at 40 °C and then at 60 °C for 8 h to give 213 mg (79%, 0.381 mmol) of ivabradine oxalate.

| | |
|---|---|
| **IR** | : 2943.6, 2834.3, 1718.9, 1643.5, 1519.8, 1485.4, 1464.7, 1304.5, 1248.0, 1221.9, 1210.0, 1183.8, 1107.0, 1071.2, 862.9, 832.4, 700.6, 178.8 |
| **DSC** | : 101.3 °C (-38.2 J/g) |
| **HPLC** | : 96.3% |

Ivabradine oxalate III is characterized by an XRD pattern having peaks at 16.9 ± 0.2, 17.5 ± 0.2, 20.9 ± 0.2, 23.1 ± 0.2, and 32.3 ± 0.2 degrees 2-theta.

### Example 13

The stability of ivabradine adipate in comparison to ivabradine hydrochloride form I was investigated at different temperatures and humidities in open or closed containers for different storage times. The results are summarized in the following table.

**Table: Stabilty of Ivabradine adipate versus Ivabradine HCl, form I**

| Temp./humidity, | HCl | Adipate |
|---|---|---|
| Container, days | Form I | |
| 25°C/60% | unchanged | |
| closed, 33d | | |
| 25°C/60% | | unchanged |
| closed, 57d | | |
| 25°C/60% | β + unident. | |
| open, 33d | cryst. phase | |
| 25°C/60% | | unchanged |
| open, 57d | | |
| 30°C/65% | unchanged | |
| closed, 33d | | |
| 30°C/65% | | unchanged |
| closed, 57d | | |
| 30°C/65% | β | |
| open, 33d | | |
| 30°C/65% | | unchanged |
| pen, 57d | | |
| 40°C/75% | unchanged | |
| closed, 33d | | |
| 40°C/75% | | unchanged |
| closed, 57d | | |
| 40°C/75% | β | |
| open, 33d | | |
| 40°C/75% | | unchanged |
| open, 57d | | |

Ivabradine adipate according to the present invention is stable at various conditions. The Ivabradine HCl form I undergoes phase transition into Ivabradine HCl, form beta, in particular in open containers.

## Claims

1. Pharmaceutical composition with modified release, comprising ivabradine adipate and a combination of a water-insoluble and a water-soluble excipient, wherein the water-soluble excipient has a water solubility of ≥ 33 mg/ml at 25°C in water and the water-insoluble excipient has a solubility of < 33 mg/ml at 25°C in water.

2. Pharmaceutical composition according to claim 1, wherein the water-soluble excipient has a water solubility of ≥ 100 mg/ml at 25°C in water and/or the water-insoluble excipient has a solubility of < 10 mg/ml at 25°C in water.

3. Pharmaceutical composition according to any one of the preceding claims, wherein the water-soluble excipient is a water-soluble polymeric cellulose derivative, preferably hydroxypropylmethylcellulose, hydroxyethylcellulose, hydroxypropylcellulose, sodium carboxymethylcellulose, cellulose acetate phthalate and/or hydroxypropylmethylcellulose phthalate, or polyvinylpyrrolidone or a copolymer thereof or PEG.

4. Pharmaceutical composition according to any of the preceding claims, wherein the water-insoluble excipient is a water-insoluble polymeric cellulose derivative, preferably ethylcellulose, ethylhydroxyethylcellulose, microcrystallinecellulose or cellulose powder, or polyvinylacetate, starch, glyceryl palmitate stearate or an ion exchange resin, preferably polacrilin potassium, sodium polystyrenesulphonate and/or colestyramine resinate.

5. Pharmaceutical composition according to any of the preceding claims, which is present as a tablet.

6. Pharmaceutical composition according to any of claims 1 to 5, the active substance being present in a matrix which controls the release, and the matrix containing the combination of a water-soluble and a water-insoluble excipient.

7. Pharmaceutical composition according to claim 6, the matrix comprising ivabradine adipate, water-soluble HPMC, lactose, microcrystalline cellulose, a glidant and a release agent, wherein the release agent is colloidal silica or magnesium stearate.

8. Pharmaceutical composition according to any of claims 1 to 5, comprising a core which has the active substance as a mixture with a combination of a water-soluble and a water-insoluble excipient and a coating which controls the release of the active substance.

9. Pharmaceutical composition according to claim 8, the pharmaceutical composition having a core consisting of ivabradine adipate, microcrystalline cellulose, sorbitol, a glidant and a release agent, and a polymethacrylate coating on the core, wherein the release agent is colloidal silica or magnesium stearate.

10. Process for the preparation of a pharmaceutical composition according to any of claims 1 to 9, comprising the steps of mixing ivabradine adipate with a combination of a water-insoluble and a water-soluble excipient and optionally pressing the composition to give a tablet.

11. Salt of ivabradine with adipic acid.

12. Non-therapeutic use of a combination of a water-insoluble and a water-soluble excipient in a pharmaceutical composition containing ivabradine adipate for controlling the release of the active substance, wherein the water-soluble excipient has a water solubility of ≥ 33 mg/ml at 25°C in water and the water-insoluble excipient has a solubility of < 33 mg/ml at 25°C in water.

13. Non-therapeutic use of a combination of a water-soluble and a water-insoluble excipient as a mixture with the active substance ivabradine adipate in a pharmaceutical composition with modified release, for stabilizing the active substance, wherein the water-soluble excipient has a water solubility of ≥ 33 mg/ml at 25°C in water and the water-insoluble excipient has a solubility of < 33 mg/ml at 25°C in water.

## Patentansprüche

1. Pharmazeutische Zusammensetzung mit modifizierter Freisetzung, umfassend Ivabradinadipat und eine Kombination aus einem wasserunlöslichen und einem wasserlöslichen Hilfsstoff, wobei der wasserlösliche Hilfsstoff eine Wasserlöslichkeit von ≥ 33 mg/ml bei 25 °C in Wasser hat und der wasserunlösliche Hilfsstoff eine Löslichkeit von < 33 mg/ml bei 25 °C in Wasser hat.

2. Pharmazeutische Zusammensetzung nach Anspruch 1, wobei der wasserlösliche Hilfsstoff eine Wasserlöslichkeit von ≥ 100 mg/ml bei 25 °C in Wasser und/oder der wasserunlösliche Hilfsstoff eine Löslichkeit von < 10 mg/ml bei 25 °C in Wasser hat.

3. Pharmazeutische Zusammensetzung nach einem der vorstehenden Ansprüche, wobei der wasserlösliche Hilfsstoff ein wasserlösliches polymeres Cellulosederivat, bevorzugt Hydroxypropylmethylcellulose, Hydroxyethylcellulose, Hydroxypropylcellulose, Natriumcarboxymethylcellulose, Celluloseacetatphthalat und/oder Hydroxypropylmethylcellulosephthalat, oder Polyvinylpyrrolidon oder ein Copolymer davon oder PEG ist.

4. Pharmazeutische Zusammensetzung nach einem der vorstehenden Ansprüche, wobei der wasserunlösliche Hilfsstoff ein wasserunlösliches polymeres Cellulosederivat, bevorzugt Ethylcellulose, Ethylhydroxyethylcellulose, mikrokristalline Cellulose oder Cellulosepulver, oder Polyvinylacetat, Stärke, Glycerylpalmitatstearat oder ein Ionenaustauschharz, bevorzugt Polacrilinkalium, Natriumpolystyrolsulfonat und/oder Cholestyraminresinat, ist.

5. Pharmazeutische Zusammensetzung nach einem der vorstehenden Ansprüche, die als eine Tablette vorliegt.

6. Pharmazeutische Zusammensetzung nach einem der Ansprüche 1 bis 5, wobei die aktive Substanz in einer Matrix vorliegt, die die Freisetzung steuert, und die Matrix die Kombination aus einem wasserlöslichen und einem wasserunlöslichen Hilfsstoff enthält.

7. Pharmazeutische Zusammensetzung nach Anspruch 6, wobei die Matrix Ivabradinadipat, wasserlösliche HPMC, Lactose, mikrokristalline Cellulose, ein Gleitmittel und ein Trennmittel umfasst, wobei das Trennmittel kolloidales Siliciumdioxid oder Magnesiumstearat ist.

8. Pharmazeutische Zusammensetzung nach einem der Ansprüche 1 bis 5, umfassend einen Kern, der die aktive Substanz als ein Gemisch mit einer Kombination aus einem wasserlöslichen und einem wasserunlöslichen Hilfsstoff aufweist, und eine Beschichtung, die die Freisetzung der aktiven Substanz steuert.

9. Pharmazeutische Zusammensetzung nach Anspruch 8, wobei die pharmazeutische Zusammensetzung einen Kern, bestehend aus Ivabradinadipat, mikrokristalliner Cellulose, Sorbitol, einem Gleitmittel und einem Trennmittel, und eine Polymethacrylat-Beschichtung auf dem Kern aufweist, wobei das Trennmittel kolloidales Siliciumdioxid oder Magnesiumstearat ist.

10. Verfahren zur Herstellung einer pharmazeutischen Zusammensetzung nach einem der Ansprüche 1 bis 9, umfassend die Schritte des Mischens von Ivabradinadipat mit einer Kombination aus einem wasserunlöslichen und einem wasserlöslichen Hilfsstoff und gegebenenfalls des Pressens der Zusammensetzung unter Erhalt einer Tablette.

11. Salz von Ivabradin mit Adipinsäure.

12. Nicht-therapeutische Verwendung einer Kombination aus einem wasserunlöslichen und einem wasserlöslichen Hilfsstoff in einer pharmazeutischen Zusammensetzung, enthaltend Ivabradinadipat, zur Steuerung der Freisetzung der aktiven Substanz, wobei der wasserlösliche Hilfsstoff eine Wasserlöslichkeit von ≥ 33 mg/ml bei 25 °C in Wasser hat und der wasserunlösliche Hilfsstoff eine Löslichkeit von < 33 mg/ml bei 25 °C in Wasser hat.

13. Nicht-therapeutische Verwendung einer Kombination aus einem wasserlöslichen und einem wasserunlöslichen Hilfsstoff als ein Gemisch mit der aktiven Substanz Ivabradinadipat in einer pharmazeutischen Zusammensetzung mit modifizierter Freisetzung zur Stabilisierung der aktiven Substanz, wobei der wasserlösliche Hilfsstoff eine Wasserlöslichkeit von
≥ 33 mg/ml bei 25 °C in Wasser hat und der wasserunlösliche Hilfsstoff eine Löslichkeit von < 33 mg/ml bei 25 °C in Wasser hat.

## Revendications

1. Composition pharmaceutique à libération modifiée, comprenant de l'adipate d'ivabradine et une combinaison d'un excipient soluble dans l'eau et un insoluble dans l'eau, dans laquelle l'excipient soluble dans l'eau a une solubilité dans l'eau de ≥ 33mg/ml à 25°C dans l'eau et l'excipient insoluble dans l'eau a une solubilité de < 33mg/ml à 25°C dans l'eau.

2. Composition pharmaceutique selon la revendication 1, dans laquelle l'excipient soluble dans l'eau a une solubilité dans l'eau de ≥ 100 mg/ml à 25°C dans l'eau et /ou l'excipient insoluble dans l'eau a une solubilité de <10 mg/ml à 25°C dans l'eau.

3. Composition pharmaceutique selon l'une quelconque des revendications précédentes, dans laquelle l'excipient soluble dans l'eau est un dérivé de cellulose polymère soluble dans l'eau, de préférence l'hydroxypropylméthylcellulose, l'hydroxyéthylcellulose, l'hydroxypropylcellulose, la carboxyméthylcellulose de sodium, l'acétate phtalate de cellulose et/ou le phtalate d'hydroxypropylméthylcellulose, ou la polyvinylpyrrolidone ou un de leur copolymère ou le PEG.

4. Composition pharmaceutique selon l'une quelconque des revendications précédentes, dans laquelle l'excipient insoluble dans l'eau est un dérivé de cellulose polymère insoluble dans l'eau, de préférence l'éthylcellulose, l'éthylhydroxyéthylcellulose, la cellulose microcristalline ou la poudre de cellulose, ou le polyvinylacétate, l'amidon, le stéarate et palmitate de glycéryle ou une résine échangeuse d'ions, de préférence la polacriline de potassium, le polystyrène sulfonate de sodium et/ou le résinate de colestyramine.

5. Composition pharmaceutique selon l'une quelconque des revendications précédentes, qui est présente sous forme d'un comprimé.

6. Composition pharmaceutique selon l'une quelconque des revendications 1 à 5, la substance active étant présente dans une matrice qui contrôle la libération, et la matrice contenant la combinaison d'un excipient soluble dans l'eau et un insoluble dans l'eau.

7. Composition pharmaceutique selon la revendication 6, la matrice comprenant de l'adipate d'ivabradine, du HPMC soluble dans l'eau, du lactose, de la cellulose microcristalline, un agent de glissement et un agent de libération, dans laquelle l'agent de libération est de la silice colloïdale ou du stéarate de magnésium.

8. Composition pharmaceutique selon l'une quelconque des revendications 1 à 5, comprenant un noyau qui a la substance active comme un mélange avec une combinaison d'un excipient soluble dans l'eau et un insoluble dans l'eau et un revêtement qui contrôle la libération de la substance active.

9. Composition pharmaceutique selon la revendication 8, la composition pharmaceutique ayant un noyau consistant en de l'adipate d'ivabradine, la cellulose microcristalline, le sorbitol, un agent de glissement et un agent de libération, et un revêtement de polyméthacrylate sur le noyau, dans laquelle l'agent de libération est de la silice colloïdale ou du stéarate de magnésium.

10. Procédé pour la préparation d'une composition pharmaceutique selon l'une quelconque des revendications 1 à 9, comprenant les étapes de mélanger de l'adipate d'ivabradine avec une combinaison d'un excipient soluble dans l'eau et un insoluble dans l'eau et éventuellement presser la composition pour donner un comprimé.

11. Sel d'ivabradine avec de l'acide adipique.

12. Usage non-thérapeutique d'une combinaison d'un excipient insoluble dans l'eau et un soluble dans l'eau dans une combinaison pharmaceutique contenant de l'adipate d'ivabradine pour contrôler la libération de la substance active, dans laquelle l'excipient soluble dans l'eau a une solubilité dans l'eau de ≥ 33mg/ml à 25°C dans l'eau et l'excipient insoluble dans l'eau a une solubilité de < 33mg/ml à 25°C dans l'eau.

13. Usage non-thérapeutique d'une combinaison d'un excipient soluble dans l'eau et un insoluble dans l'eau comme un mélange avec la substance active d'adipate d'ivabradine dans une composition pharmaceutique avec libération modifiée, pour stabiliser la substance active, dans laquelle l'excipient soluble dans l'eau a une solubilité dans l'eau de ≥ 33mg/ml à 25°C dans l'eau et l'excipient insoluble dans l'eau a une solubilité de < 33mg/ml à 25°C dans l'eau.
